(19) Europäisches Patentamt
European Patent Office
Office européen des brevets

(11) Veröffentlichungsnummer: **0 461 499 A2**

# (12) EUROPÄISCHE PATENTANMELDUNG

(21) Anmeldenummer: **91108983.7**

(51) Int. Cl.5: **A61K 31/71**

(22) Anmeldetag: **01.06.91**

(30) Priorität: **14.06.90 DE 4019024**

(43) Veröffentlichungstag der Anmeldung:
**18.12.91 Patentblatt 91/51**

(84) Benannte Vertragsstaaten:
**AT BE CH DE DK ES FR GB GR IT LI LU NL SE**

(71) Anmelder: **BAYER AG**

**W-5090 Leverkusen 1 Bayerwerk(DE)**

(72) Erfinder: **Müller, Hartwig, Dr.**
**Steinstrasse 15**
**W-5620 Velbert 15(DE)**
Erfinder: **Bischoff, Erwin, Dr.**
**Pahlkestrasse 73**
**W-5600 Wuppertal(DE)**
Erfinder: **Fugmann, Burkhard, Dr.**
**Gerhardtstrasse 47**
**W-4030 Ratingen 1(DE)**
Erfinder: **Weber, Karlheinz, Dr.**
**Auf dem Scheidt 17**
**W-5600 Wuppertal(DE)**
Erfinder: **Frobel, Klaus, Dr.**
**Paul-Ehrlich-Strasse 9**
**W-5600 Wuppertal(DE)**
Erfinder: **Rosen, Bruno, Dr.**
**Marienburger Strasse 65**
**W-5603 Wülfrath(DE)**
Erfinder: **Grützmann, Rudi, Dr.**
**Helsinki-Strasse 20**
**W-5650 Solingen(DE)**
Erfinder: **Karmann, Günther, Dr.**
**Hoppersheiderweg 26**
**S-5060 Bergisch-Gladbach 2(DE)**
Erfinder: **Kohlsdorfer, Christian, Dr.**
**Franz-Stryck-Strasse 26**
**W-5054 Erftstadt(DE)**

(54) **Verwendung der Efomycine A,E und G als entzündungshemmende Mittel.**

(57) Die vorliegende Erfindung betrifft die Verwendung der bekannten Naturstoffe Efomycin A, E und G der allgemeinen Formel

**EP 0 461 499 A2**

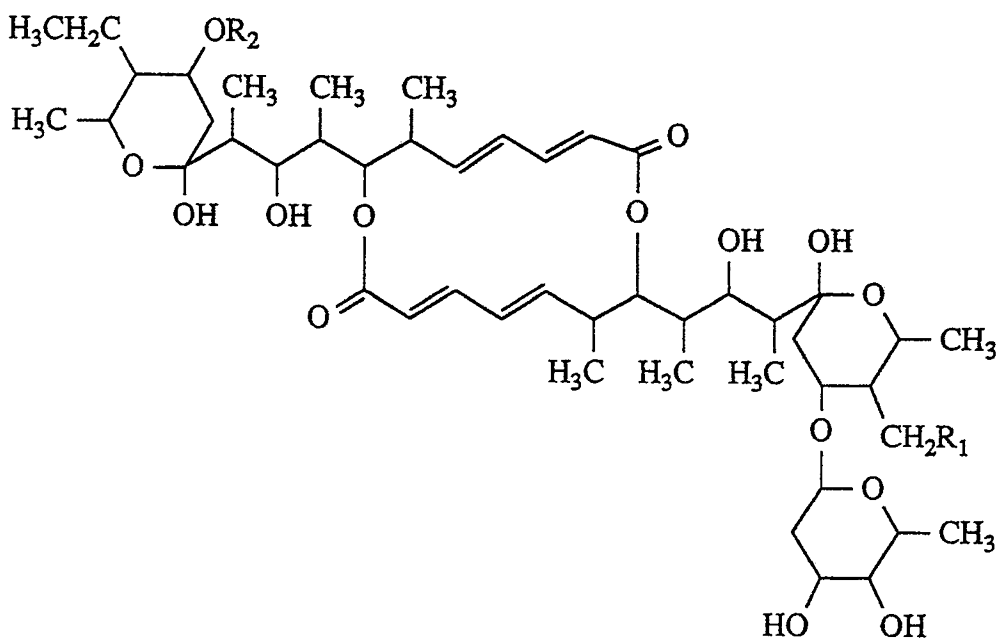

(I)

in welcher $R_1$ und $R_2$ die in der Beschreibung angegebene Bedeutung haben, zur Behandlung von akuten und chronischen Entzündungen, insbesondere ihre Verwendung als Arzneimittel bei der Myokardinfarkttherapie.

Die vorliegende Erfindung betrifft die Verwendung der bekannten Naturstoffe Efomycin A, E und G zur Behandlung von akuten und chronischen Entzündungen, insbesondere ihre Verwendung als Arzneimittel bei der Myokardinfarkttherapie.

Es ist bereits bekannt, daß die Naturstoffe Efomycin A, E und G eine antibakterielle Wirkung auf Mikroorganismen im Pansen besitzen und als Leistungs- und Wachstumsförderer in der Tiermedizin eingesetzt werden können [vgl. EP 197 360 und EP 236 894].

Außerdem ist bekannt, daß Efomycin E nach Röntgenstrukturuntersuchungen identisch ist mit dem Antibiotikum Elaiophylin [vgl. Helv. Chim. Acta 64, 407-424 (1981); 65, 262-267], das unter dem Namen Azalomycin B im U.S.-Patent 30 76 746 mit antibiotischer und antimikrobieller Wirkung publiziert wurde.

Es wurde nun gefunden, daß die Naturstoffe Efomycin A, E und G der allgemeinen Formel (I),

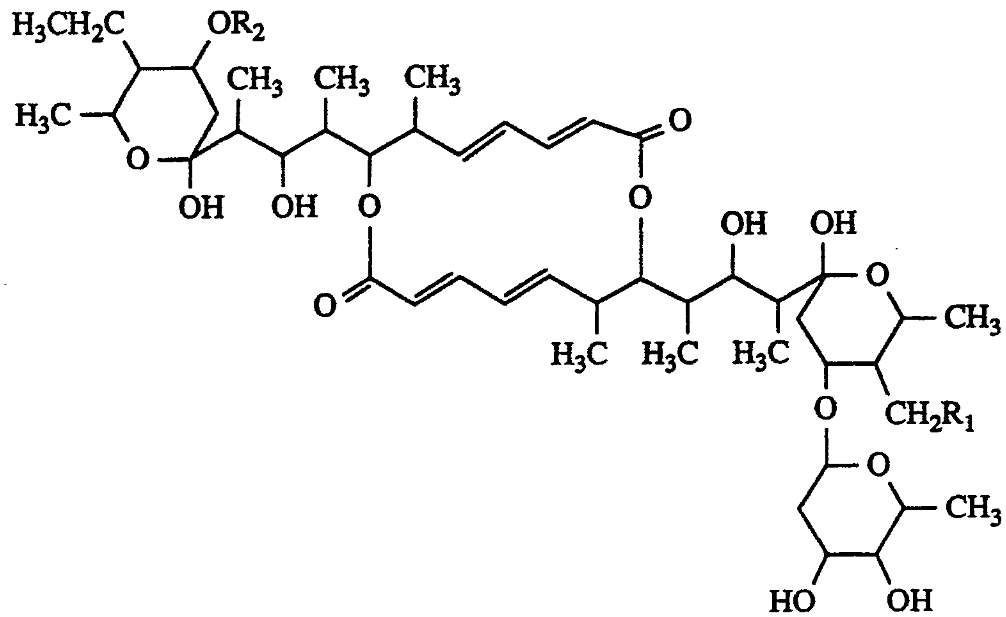

(I)

in welcher

$R^1$ für Methyl steht
und
$R^2$ für einen Rest der Formel

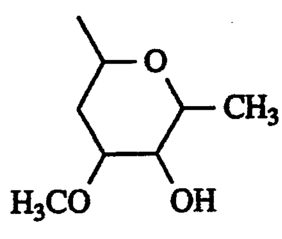

steht (Efomycin A),

oder

$R^1$ für Methyl steht und
$R^2$ für einen Rest der Formel

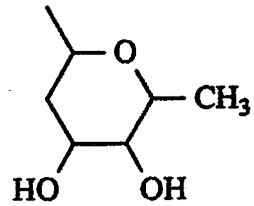

steht (Efomycin E),

oder

$R^1$ für Wasserstoff steht

und

$R^2$ für einen Rest der Formel

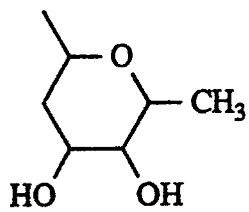

steht (Efomycin G),

neben den bekannten leistungs- und wachstumsfördernden, antibiotischen und antimikrobiellen Aktivitäten überraschenderweise auch eine außerordentlich stark hemmende Wirkung auf die Adhäsion von Leukozyten bei akuten und chronischen Entzündungen sowie akuten ischämischen Zuständen besitzen.

Die detaillierten chemischen und physikalischen Charakterisierungen und die Verfahren zur Herstellung der erfindungsgemäß zu verwendenden Verbindungen der Formel (I) sind bekannt [vgl. EP 197 360 und EP 236 894].

Die Verbindungen der Formel (I) können als Wirkstoffe in Arzneimitteln bei akuten und chronischen entzündlichen Prozessen und/oder einer akuten Durchblutungsstörung (Ischämie) eingesetzt werden.

Ihre Wirkung besteht zunächst in der Verminderung oder vollständigen Inhibierung der Adhäsion von Leukozyten an vaskulärem Endothel und der damit verbundenen Verminderung oder Abschwächung der zeitlich nachgeordneten unerwünschten Prozesse, wie die Sauerstoffradikalfreisetzung (durch polymorphkernige neutrophile Leukozyten) oder Schaumzellbildung (Monozyten).

Sie sind bevorzugt zur Behandlung und Verhütung der Gewebsnekrose nach akutem Myokardinfarkt geeignet Außerdem können sie zur Behandlung und Verhütung von akuten und chronischen, nicht infektionsbedingten Entzündungen der Atemwege, wie allergischem Asthma, sowie bei Rheuma, Arteriosklerose, Arthrose und Entzündungen des Gastrointestinaltraktes eingesetzt werden.

Anwendungsbeispiel 1:

Die erfindungsgemäßen Substanzen hemmen die Adhäsion von aktivierten Leukozyten an Plastikoberflächen.

Der Test wird in einer Mikrotiterplatte durchgeführt. Jede Vertiefung wird 30 min. mit phosphatgepufferter Saline (PBS), in der 1% Rinderserumalbumin gelöst ist, inkubiert und danach zweimal mit PBS gewaschen. Aliquots von 100 µl RPMI 1640 (RPMI-1640 Medium with Glutamine Nr. 041-01875 M Gibco Limited GB] Medium, das 10% foetales Kälberserum (FCS) und die zu prüfenden Substanzen enthält, werden in die Vertiefungen pipettiert und die Platte bei 37° C vorinkubiert. Alle weiteren Inkubationen wurden unter diesen Bedingungen durchgeführt. Die PMN [Polymorphkernige neutrophile Leuzoyten, isoliert nach English et al., J. Immunol. Methods 5, 249-252 (1974)] werden in RPMI 1640 Medium suspendiert und 2 x $10^4$ Zellen in 50 µl in jede Vertiefung pipettiert. Nach einer Inkubation von 10 min. werden die Platten 30 sec. geschüttelt und die Zellen mit 50 µl Phorbolmyristatacetat(Endkonzentration $10^{-6}$ mol/l) aktiviert Danach wird 30 sec. geschüttelt, 1 Stunde inkubiert, erneut 30 sec. geschüttelt und die nicht adhärenten Zellen durch zweimaliges Waschen mit mit je 300 µl PBS entfernt. Die adhärenten Zellen werden durch Zugabe von 300 µl 5% Cetyltrimethylammoniumbromid in 5 mM Phosphatpuffer pH 7,4 lysiert, indem sie

10 min. bei Raumtemperatur geschüttelt werden.

Die quantitative Bestimmung der PMN erfolgt über die Messung der Myeloperoxidaseaktivität. Dazu werden 270 µl eines Gemisches aus Phosphatpuffer 50 mM pH 6,0, ortho-Dianisidin, 0,23 mg/ml und $H_2O_2$ $10^{-4}$ mol/l zugegeben. Der Anstieg der Extinktion bei 460 nm wird 4 mal im Abstand von 2 min. gemessen.

Tabelle 1

| | Hemmung der Adhäsion durch Efomycin A, E und G [%] |
|---|---|
| Kontrolle | 0 |
| Efomycin E $5 \times 10^{-6}$ mol/l | 93 |
| Efomycin E $10^{-6}$ mol/l | 62 |
| Efomycin G $5 \times 10^{-6}$ mol/l | 95 |
| Efomycin G $10^{-6}$ mol/l | 68 |
| Efomycin A $5 \times 10^{-6}$ mol/l | 60 |
| Efomycin A $10^{-6}$ mol/l | 25 |

Anwendungsbeispiel 2

Die erfindungsgemäßen Substanzen inhibieren auch die Adhäsion nicht stimulierter PMNL an aktiviertes Endothel.

1. Präparation der Zellpopulation

Humane Endothelzellen wurden aus Nabelschnüren durch Collagenase-Behandlung gewonnen und über 2-5 Passagen in Medium mit essentiellen Zusätzen wie Heparin und Endothelial Cell Growth Supplement kultiviert. Anschließend wurden die Zellen durch Trypsin abgelöst, in einer Dichte von 1-5 x $10^4$ Zellen in Mikrotiterplatten ausgesät und bis zum Erreichen von Konfluenz weitere zwei Tage inkubiert.

Humane polymorphkernige Granulozyten (PMN) wurden aus Frischblut über Sedimentation in 6%

Dextransulfat gefolgt von Zentrifugation über ein Ficoll-Kissen und hypotonischer Lyse der Erythrozyten präpariert.

2. Adhäsionstest

Unstimulierte PMN in einer Konzentration von 2,5-5 x $10^5$ Zellen/Vertiefung sowie die in DMSO gelösten Prüfsubstanzen wurden zu den Endothelzellen hinzugefügt. Nach einer 30-minütigen Inkubation (37° C, 10% $CO_2$) wurden unspezifisch gebundene PMN durch Zentrifugation der umgedrehten Platte entfernt [vgl. Charo et al., Blood, 65, 473-479 (1985). Die Zahl der an Endothelzellen gebundenen PMN wurde über die Aktivität des in PMN, nicht jedoch in Endothelzellen vorkommenden Enzyms Myeloperoxidase bestimmt.

3. Wirkung der Substanzen Efo E und Efo G

Die Prüfsubstanzen Efo E und Efo G wurden in dem beschriebenen Meßsystem in einer Endkonzentration von 5 x $10^{-6}$ Mol/l eingesetzt. Die Lösemittelkonzentration in der Kultur betrug dann 0,1% DMSO.

Die PMN-Adhäsion am Endothel wurde durch Efo E und Efo G gegenüber der Kontrolle ohne Zugabe von Substanz um 20 und 27% reduziert (n = 6, $p < 0,01$ bzw. $p < 0,001$ nach t-Test). Die Lösemittelkontrolle mit 0,1% DMSO ergab keine signifikante Veränderung in der Zahl der adhärenten PMN.

Anwendungsbeispiel 3

Anstatt Endothelzellen wurden HeLa 229-Zellen (ATCC CCL 2.1) verwendet, die in einem Kulturgefäß an der Oberfläche adhäriert waren. Die Adhäsion humaner PMN an diese Zellen kann durch die Efomycine E und G dosisabhängig inhibiert werden.

Tabelle 2

Hemmung der Adhäsion von Leukozyten an HeLa-Zellen durch die Efomycine E und G.

| | |
|---|---|
| Kontrolle | 0[%] |
| Efomycin E | |
| 1 x $10^{-5}$ Mol/L | 57 |
| 3,3 x $10^{-6}$ Mol/L | 45,4 |
| 1,1 x $10^{-6}$ Mol/L | 21,8 |
| 3,7 x $10^{-7}$ Mol/L | 13,3 |
| 1 x $10^{-7}$ Mol/L | 3,0 |
| Efomycin G | |
| 1 x $10^{-5}$ Mol/L | 49,5 |
| 3,3 x $10^{-6}$ Mol/L | 32,6 |
| 1,1 x $10^{-6}$ Mol/L | 32,1 |
| 3,7 x $10^{-7}$ Mol/L | 4,4 |

Anwendungsbeispiel 4

Im Modell der Hamsterbackentasche kann die Adhäsion von Leukozyten unter ischämischen und nichtischämischen Bedingungen in Mikrogefäßen quantifiziert werden. Unter nichtischämischen Bedingungen wird die Adhäsion von Efomycin G bei 1,0 mg/kg i.v. um 40% und unter ischämischen Bedingungen um 60% gegenüber Kontrolle vermindert. Bei dieser Dosis ist kein Effekt auf den Blutdruck zu beobachten

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter

Verwendung inerter nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös.

Im allgemeinen hat es sich als vorteilhaft erwiesen, bei intravenöser Applikation Mengen von etwa 0,001 bis 1 mg/kg, vorzugsweise etwa 0,01 bis 0,5 mag/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. dem Intervall, zu welchen die Verabreichung erfolgt. So kann es in einigen Fällen ausreichend sein, mit weniger als der vorgenannen Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

**Patentansprüche**

1. Verwendung von Verbindungen der allgemeinen Formel (I)

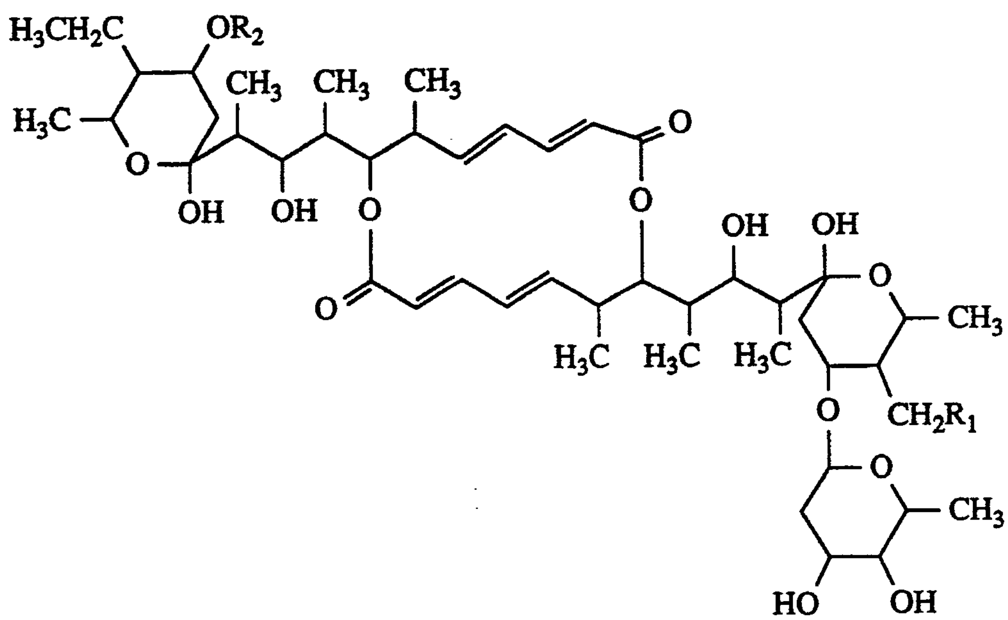

(I)

in welcher

$R^1$ für Methyl steht

und

$R^2$ für einen Rest der Formel

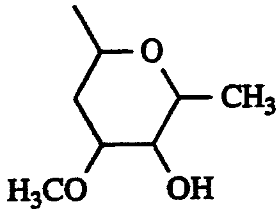

steht (Efomycin A),

oder

$R^1$ für Methyl steht

und

$R^2$ für einen Rest der Formel

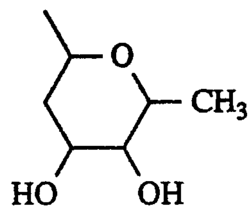

steht (Efomycin E),

oder

$R^1$ für Wasserstoff steht

und

$R^2$ für einen Rest der Formel

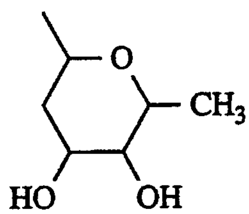

steht (Efomycin G),

bei der Herstellung von Arzneimitteln zur Behandlung von akuten und chronischen Entzündungen sowie von ischämischen Zuständen.

2. Verwendung von Verbindungen der Formel (I) gemäß Anspruch 1 bei der Herstellung von Arzneimitteln zur Behandlung und Verhütung von Entzündungen der Atemwege und des Gastrointestinaltraktes.

3. Verwendung von Verbindungen der allgemeinen Formel (I) gemäß Anspruch 1 bei der Herstellung von Arzneimitteln für die Behandlung von Gewebsnekrosen nach akutem Myokardinfarkt, Rheuma, Arteriosklerose und Arthrose.

4. Verwendung von Verbindungen der allgemeinen Formel (I) gemäß Anspruch 1 bei der Herstellung von Arzneimitteln zur Verminderung oder vollständigen Inhibierung der Adhäsion von Leukozyten an vaskulärem Endothel.

5. Verwendung von Verbindungen der allgemeinen Formel (I) gemäß Anspruch 1 bei der Bekämpfung von akuten und chronischen endzündlichen Prozessen sowie von akuten Durchblutungsstörungen.